# FASCICULE DE BREVET EUROPEEN

(11) **EP 2 925 210 B1**
(45) Date de publication et mention de la délivrance du brevet: **08.01.2020**
(21) Numéro de dépôt: 13820788.1
(22) Date de dépôt: 27.11.2013
(51) Int. Cl.: A61B 3/113, A61B 3/00, A61B 5/16

(54) **PROCÉDÉ ET SYSTÈME DE COLLECTE D'UN PARAMÈTRE DE SACCADE DE RETOUR LORS D'UN TEST VISUEL, ET UTILISATION POUR DIAGNOSTIQUER UN TROUBLE OCULOMOTEUR**
VERFAHREN UND SYSTEM ZUR ERFASSUNG EINES RÜCKWÄRTSSAKKADENPARAMETERS WÄHREND EINES AUGENTESTS UND VERWENDUNG ZUR DIAGNOSE EINER BLICKMOTORISCHEN ERKRANKUNG
METHOD AND SYSTEM FOR COLLECTING A RETURN SACCADE PARAMETER DURING A VISUAL TEST, AND USE FOR DIAGNOSING AN OCULOMOTOR DISORDER

(30) Priorité: 30.11.2012 FR 1261521
(43) Date de publication de la demande: 07.10.2015
(73) Titulaire: Suricog, 75015 Paris (FR)
(72) Inventeur: SEASSAU, Magali, 75012 Paris (FR)
(74) Mandataire: Nony
(86) Numéro de dépôt international: PCT/FR2013/052879
(87) Numéro de publication internationale: WO 2014/083281

(56) Documents cités:
- WO-A1-2004/112598
- WO-A1-2013/102768
- US-A- 3 906 644
- US-A- 4 528 989
- US-A- 4 838 681
- US-A- 4 961 640
- OLIVIER A COUBARD ET AL: "Saccades during symmetrical vergence", GRAEFE'S ARCHIVE FOR CLINICAL AND EXPERIMENTAL OPHTHALMOLOGY ; INCORPORATING GERMAN JOURNAL OF OPHTHALMOLOGY, SPRINGER, BERLIN, DE, vol. 246, no. 4, 22 novembre 2007 (2007-11-22), pages 521-536, XP019589845, ISSN: 1435-702X

## Description

### DOMAINE DE L'INVENTION

La présente invention concerne le domaine de la collecte de données lorsqu'un sujet est soumis à un test visuel impliquant au moins un retour à la ligne d'au moins un œil du sujet. L'invention concerne plus particulièrement un procédé visant à la collecte de telles données, un système correspondant, ainsi que l'utilisation d'un tel système pour diagnostiquer un trouble oculomoteur, de préférence un trouble oculomoteur impliqué dans la dyslexie.

### CONTEXTE DE L'INVENTION

Le procédé et le système peuvent notamment aider à la détection d'un trouble ou anormalité oculomotrice chez un sujet, pouvant être à l'origine d'une dyslexie.

Le trouble oculomoteur, c'est-à-dire une anormalité du mouvement des yeux, peut se manifester dans certaines maladies, par exemple le strabisme ou la dyslexie de surface qui est un type principal de dyslexie, une autre forme principale de dyslexie étant la dyslexie phonologique par laquelle un sujet confond des lettres ou des sons.

La dyslexie de surface peut intégrer une dyspraxie du regard, c'est-à-dire d'une mauvaise coordination binoculaire d'un sujet. La coordination binoculaire reflète la coordination droite-gauche entre les deux yeux du sujet. En d'autres termes, il s'agit du mouvement relatif des deux yeux pendant une même tâche, autrement dénommé la *« vergence* » dans la littérature anglo-saxonne, introduisant, en cas de dyspraxie, un décalage spatial entre les deux yeux pouvant se mesurer en degrés.

Les publications « Dyslexic children are confronted with unstable binocular fixation while reading » (S. Jainta et al., 2011) (voir page 1, colonne de droite, lignes 3 à 15) et « The binocular coordination of eye movements during reading in children and adults », H. Blythe at al., 2006 (voir section 1.1 et p.3899, colonne de droite, lignes 49 à 55) font appel à une analyse de cette coordination binoculaire lors de tests de lecture.

De nombreux tests ont été mis en place pour tenter de détecter le plus tôt possible la dyslexie de surface chez les jeunes enfants.

Les travaux de Rayner (1978) et Pavlidis (1981) ont notamment montré que les enfants atteints d'une telle dyslexie présentaient de sérieux problèmes de lecture, caractérisés notamment par un grand nombre de fixations régressives (c'est-à-dire vers la gauche pour un texte se lisant de gauche à droite, par opposition aux fixations progressives), par des saccades progressives plus courtes (les saccades représentant le déplacement des yeux d'un mot à un autre) et par des fixations plus longues que des lecteurs normaux du même âge.

Des tests de lecture ont ainsi été développés, comme évoqués dans ces deux publications susvisées, et l'exploitation de paramètres a été affinée.

De même, des tests de recherche visuelle, notamment des occurrences d'une lettre au sein d'une suite de lettres, ont été développés, comme évoqués dans les publications *"*The eye movements of dyslexic children during reading and visual search: Impact of the visual attention span" de C.Prado, M. Dubois et S. Valdois (Vision Research 47 pp.2521-2530, 2007) et *"*Immaturity of binocular saccade coordination in dyslexic children: evidence from a reading and visual search study" de Maria Pia Bucci, Naziha Nassibi, Christophe-Loic Gerard, Emmanuel Bui-Quoc, Magali Seassau (PlosOne 7(3), e33458), à partir desquels des paramètres ont été exploités.

Ces tests sont des tests visuels impliquant au moins un retour à la ligne d'au moins un œil du sujet, généralement plusieurs retours à la ligne. Le test de lecture consiste à lire une suite de symboles, généralement typographiques, répartie sur plusieurs lignes horizontales alors que le test de recherche visuelle consiste à rechercher et trouver les occurrences d'un symbole défini au sein de la suite de symboles.

Dans ces tests, le sujet parcourt ou balaye les symboles ligne par ligne. Un retour à la ligne correspond ainsi au déplacement de l'œil entre une fin de ligne de symboles et le début de la ligne suivante de symboles, suivant le sens de parcours ou balayage des lignes.

Dans la publication *"*The eye movements of dyslexic children during reading and visual search: Impact of the visual attention span" de C.Prado, M. Dubois et S. Valdois (Vision Research 47 pp.2521-2530, 2007), un test de lecture de texte francophone et un test de recherche visuelle d'une lettre à l'intérieur d'un paragraphe dépourvu de contenu sémantique ont été pratiqués sur des sujets enfants pour déterminer l'influence de certains paramètres dans la détection de la dyslexie. Le nombre total de fixations, la durée moyenne de fixation, le pourcentage et la durée des fixations régressives, le nombre et la durée des fixations progressives ont été calculés à partir d'enregistrements des mouvements de l'oeil droit des sujets.

Il arrive que les paramètres utilisés dans ces publications n'identifient pas avec certitude certains sujets dyslexiques.

Le document D1 (WO 2004/112598) concerne une méthode de test et d'aide au patient qui s'appuie directement sur des mesures de suivi des yeux lorsque le patient réalise des tâches optiques. Des paramètres colorimétriques sont notamment déterminés. Un certain nombre de paramètres à mesurer sont proposés.

### RESUME DE L'INVENTION

Les inventeurs ont constaté que l'utilisation d'un ou plusieurs autres paramètres bien choisis permet d'améliorer l'identification de sujets dyslexiques par test visuel, type tâche de lecture ou tâche de recherche visuelle.

Dans ce contexte, l'invention vise plus particulièrement un procédé, par exemple de collecte de données, comprenant les étapes suivantes :
soumettre le sujet à un test visuel impliquant au moins un retour à la ligne d'au moins un œil du sujet;
pendant ladite tâche, enregistrer les mouvements de l'œil du sujet à l'aide d'un dispositif de suivi de mouvements oculaires;
déterminer, à l'aide d'un dispositif de traitement numérique et dans les enregistrements obtenus, des saccades de retour composant tout ou partie de l'au moins un retour à la ligne de l'oeil du sujet;
calculer au moins un paramètre de saccade de retour à partir des enregistrements correspondant aux saccades de retour déterminées.

Des paramètres relatifs aux saccades de retour permettent en effet de révéler des cas de trouble oculomoteur, là où les paramètres classiques sont insuffisants. Ils se révèlent ainsi être une aide précieuse au dépistage de la dyslexie notamment.

Un tel paramètre de saccade de retour peut être obtenu en traitant les enregistrements des mouvements d'un ou des deux yeux du sujet, pendant le test visuel ou bien ultérieurement en l'absence du sujet, à partir des mesures réalisées précédemment.

Un ou des nouveaux paramètres de saccade de retour peuvent être utilisés de façon isolée ou en combinaison avec un ou plusieurs paramètres classiques.

Ainsi, dans un prolongement de l'invention, ce procédé de collecte de données peut être utilisé dans un procédé de détection d'une anormalité oculomotrice chez un sujet dans lequel, par exemple, outre les étapes précédentes, on compare le paramètre calculé avec au moins une valeur seuil de référence. En d'autres termes, on met en correspondance le paramètre déterminé avec au moins une valeur seuil pour déterminer une anormalité oculomotrice.

Ce procédé de détection permet d'aider à l'identification de sujets présentant une anormalité oculomotrice, par exemple, pour cause de dyslexie ou de strabisme, par l'utilisation d'un test visuel de type lecture ou recherche visuelle.

Corrélativement, l'invention vise un système comprenant:
un module de test pour soumettre le sujet à un test visuel impliquant au moins un retour à la ligne d'un œil du sujet;
un module de suivi de mouvements oculaires configuré pour enregistrer, pendant ladite tâche, les mouvements d'au moins un œil du sujet;
un module de traitement numérique configuré pour déterminer, dans les enregistrements obtenus, des saccades de retour composant tout ou partie de l'au moins un retour à la ligne de l'œil du sujet;
un module pour calculer au moins un paramètre de saccade de retour à partir des enregistrements correspondant aux saccades de retour déterminées.

Par prolongement, ce système, dédié à la collecte de données, peut s'inscrire dans un système de détection d'une anormalité oculomotrice chez un sujet dans lequel, par exemple, outre ces modules, le système de détection comprend également un module de comparaison du paramètre calculé avec au moins une valeur seuil de référence.

Le système selon l'invention présente des avantages similaires à ceux du procédé exposé ci-dessus, notamment celui d'offrir un ou plusieurs nouveaux paramètres améliorant la détection d'un trouble oculomoteur, par exemple lié à la dyslexie, lors de tests visuels.

Des caractéristiques optionnelles du procédé selon l'invention sont par ailleurs définies dans les revendications dépendantes. Le système selon l'invention peut également comprendre des moyens configurés pour mettre en œuvre ces caractéristiques optionnelles.

Notamment différents types de paramètres de saccade de retour peuvent être prévus.

Dans un mode de réalisation, l'au moins un paramètre de saccade de retour est fonction d'un nombre de saccades de retour constitutives d'un même retour à la ligne.

En particulier, l'au moins un paramètre de saccade de retour peut comprendre un nombre moyen de saccades de retour sur une pluralité de retours à la ligne.

En effet, les inventeurs ont constaté un accroissement moyen des saccades constitutives des retours à la ligne chez certains sujets atteints de troubles oculomoteurs. Les paramètres de saccade de retour ainsi définis s'avèrent particulièrement efficaces dans le dépistage de tels troubles, type dyslexie.

Dans un autre mode de réalisation, l'au moins un paramètre de saccade de retour est fonction d'un ratio entre une amplitude horizontale d'une saccade de retour donnée dans un retour à la ligne et une amplitude totale horizontale de saccades de retour constitutives du même retour à la ligne.

La notion d'horizontalité est à mette en relation avec l'horizontalité d'une ligne. Ainsi, l'amplitude horizontale correspond au trajet de la saccade considérée le long d'une ligne (donc le trajet de l'œil le long de cette ligne). Ce qui importe c'est de prendre l'amplitude selon l'orientation de la ligne et donc du retour à la ligne.

L'amplitude totale horizontale est généralement proche ou égale à la longueur de la ligne puisque les yeux du sujet reviennent au début de la ligne suivante, lors d'un retour à la ligne. Parfois cependant, des saccades de retour parasites, en ce qu'elles sont régressives (c'est-à-dire dans le sens opposé au retour à la ligne), peuvent intervenir accroissant l'amplitude totale horizontale, ici représentée par la somme des amplitudes (absolues) des saccades de retour constitutives du retour à la ligne.

Le ratio défini ci-dessus représente donc la portion de ligne parcourue par la saccade de retour considérée et donc la précision de cette saccade de retour dans le retour à la ligne.

En particulier, la saccade de retour donnée est de préférence la première saccade temporelle à l'intérieur du retour à la ligne. En d'autres termes, il s'agit de la saccade de retour qui débute à la fin de ligne qui vient d'être parcourue pour le test.

Les inventeurs ont constaté que ce ratio, en tant que paramètre de saccade de retour, s'avère également être un bon révélateur de troubles oculomoteurs chez certains sujets. En effet, les inventeurs ont constaté que la première saccade temporelle est généralement moins efficace chez un sujet atteint d'un trouble oculomoteur que chez un sujet sain.

Selon une autre caractéristique particulière, l'au moins un paramètre de saccade de retour comprend une moyenne de ratios sur une pluralité de retours à la ligne, chaque ratio pour un retour à ligne correspondant au ratio entre une amplitude horizontale de la première saccade temporelle de retour dans ledit retour à la ligne et une amplitude totale horizontale de saccades de retour constitutives du même retour à la ligne.

Cette configuration permet d'obtenir un paramètre de saccade de retour consolidé, car établi sur plusieurs lignes pour le même sujet. L'aide à la détection d'un trouble oculomoteur est ainsi accrue.

Dans un mode de réalisation particulier visant la combinaison des paramètres susmentionnés, l'au moins un paramètre de saccade de retour combine un nombre moyen de saccades de retour sur une pluralité de retours à la ligne et une moyenne de ratios sur la pluralité de retours à la ligne, chaque ratio pour un retour à ligne correspondant au ratio entre une amplitude horizontale de la première saccade temporelle de retour dans ledit retour à la ligne et une amplitude totale horizontale de saccades de retour constitutives du même retour à la ligne.

Comme montré ci-dessous, l'utilisation combinée de ces paramètres de saccade de retour moyennés sur plusieurs lignes est particulièrement efficace pour la détection d'un trouble oculomoteur chez un sujet.

Notamment, le procédé peut comprendre l'identification d'une anormalité oculomotrice dès lors que le nombre moyen de saccades de retour est supérieur à une première valeur seuil ou que la moyenne de ratios est inférieure à une deuxième valeur seuil.

Dans un mode de réalisation pouvant se combiner avec les modes de réalisation précédents, les mouvements de deux yeux du sujet sont enregistrés dans lesquels des saccades de retour sont déterminées, et le paramètre de saccade de retour comprend au moins un paramètre de disconjugaison binoculaire en amplitude représentatif d'une différence de mouvement horizontal entre les deux yeux du sujet lors d'au moins une saccade de retour déterminée.

Ce paramètre supplémentaire de disconjugaison saccadique améliore encore la détection d'un trouble oculomoteur chez un sujet.

Les inventeurs ont en effet constaté que la coordination binoculaire chez les sujets dyslexiques est altérée lors de phases de saccade, notamment pendant un retour à la ligne.

L'ensemble des paramètres de saccade de retour évoqués précédemment sont déterminés à partir des enregistrements obtenus.

Comme brièvement déjà introduit, le test visuel peut comprendre une tâche de lecture d'une suite de symboles répartie sur plusieurs lignes horizontales ou une tâche de recherche visuelle d'occurrences d'un symbole au sein de la suite de symboles. Avantageusement, il s'agit de symboles typographiques permettant de constituer des lignes de mots.

Un mode de réalisation combinant ces deux tâches peut ainsi prévoir que l'on soumet le sujet à une tâche de lecture d'une suite de symboles répartie sur plusieurs lignes horizontales et à une tâche de recherche visuelle d'occurrences d'un symbole au sein d'une suite de symboles répartie sur plusieurs lignes horizontales; et
le procédé comprend l'identification d'une anormalité oculomotrice dès lors qu'au moins un paramètre parmi
un nombre moyen de saccades de retour sur une pluralité de retours à la ligne lors de la tâche de lecture,
un moyenne de ratios sur la pluralité de retours à la ligne lors de la tâche de lecture, chaque ratio pour un retour à ligne correspondant au ratio entre une amplitude horizontale de la première saccade temporelle de retour dans ledit retour à la ligne et une amplitude totale horizontale des saccades de retour constitutives du même retour à la ligne,
un nombre moyen de saccades de retour sur une pluralité de retours à la ligne lors de la tâche de recherche visuelle,
un moyenne de ratios sur la pluralité de retours à la ligne lors de la tâche de recherche visuelle, chaque ratio pour un retour à ligne correspondant au ratio entre une amplitude horizontale de la première saccade temporelle de retour dans ledit retour à la ligne et une amplitude totale horizontale des saccades de retour constitutives du même retour à la ligne,
dépasse une valeur seuil correspondante, c'est-à-dire une valeur seuil pour chaque type de paramètre utilisé ci-dessus.

Ces quatre paramètres, éventuellement complétés du paramètre de disconjugaison binoculaire en amplitude évoqué plus haut, peuvent être associés au sein d'un même enregistrement comme résultat du procédé de collecte défini plus haut.

Une application principale de l'invention est la détection de la dyslexie, en particulier la dyslexie de surface, chez des sujets, notamment des enfants.

La collecte de paramètres de saccade de retour selon l'invention peut également être mise en œuvre dans un cadre d'évaluation de méthodes ou de systèmes curatifs visant à soigner et réduire le trouble oculomoteur chez des sujets déjà diagnostiqués dyslexiques.

### BREVE PRESENTATION DES FIGURES

D'autres particularités et avantages de l'invention apparaîtront encore dans la description ci-après, illustrée par les dessins ci-joints, dans lesquels :
- la **figure 1** illustre schématiquement un système d'aide à la détection d'une anormalité oculomotrice par test de recherche visuelle, selon l'invention ;
- la **figure 2** représente un exemple de paragraphe pour un test visuel de type lecture (à gauche) et de type recherche visuelle (à droite) adapté aux enfants de 8 à 9 ans;
- la **figure 3** représente un exemple de paragraphe pour un test visuel de type lecture (à gauche) et de type recherche visuelle (à droite) adapté aux enfants de 10 à 11 ans;
- la **figure 4** représente un autre exemple de paragraphe pour un test visuel de type lecture (à gauche) et de type recherche visuelle (à droite) adapté aux enfants de 12 à 13 ans;
- la **figure 5** illustre un enregistrement des mouvements des yeux d'un sujet sain pendant un test visuel;
- la **figure 6** illustre un enregistrement des mouvements des yeux d'un sujet dyslexique pendant le même test visuel;
- la **figure 7** montre un tableau récapitulatif des résultats de tests visuels sur un groupe de sujets enfants entre 8 et 9 ans;
- la **figure 8** montre un tableau récapitulatif des résultats de tests visuels sur un groupe de sujets enfants entre 10 et 11 ans;
- la **figure 9** montre un tableau récapitulatif des résultats de tests visuels sur un groupe de sujets enfants entre 12 et 13 ans ;
- les **figures 10a** et **10b** illustrent respectivement le comportement du nombre moyen de saccades de retour et le comportement du gain moyen de la première saccade temporelle de retour lors de tests de lecture et de recherche visuelle, entre les sujets dyslexiques de l'ensemble des groupes et les sujets sains; et
- les **figures 11a** et **11b** illustrent respectivement le comportement du nombre moyen de saccades de retour et le comportement du gain moyen de la première saccade temporelle de retour lors de tests de lecture et de recherche visuelle, entre les sujets dyslexiques et les sujets sains pris par groupes d'âges.

### DESCRIPTION DETAILLEE DE MODES DE REALISATION DE L'INVENTION

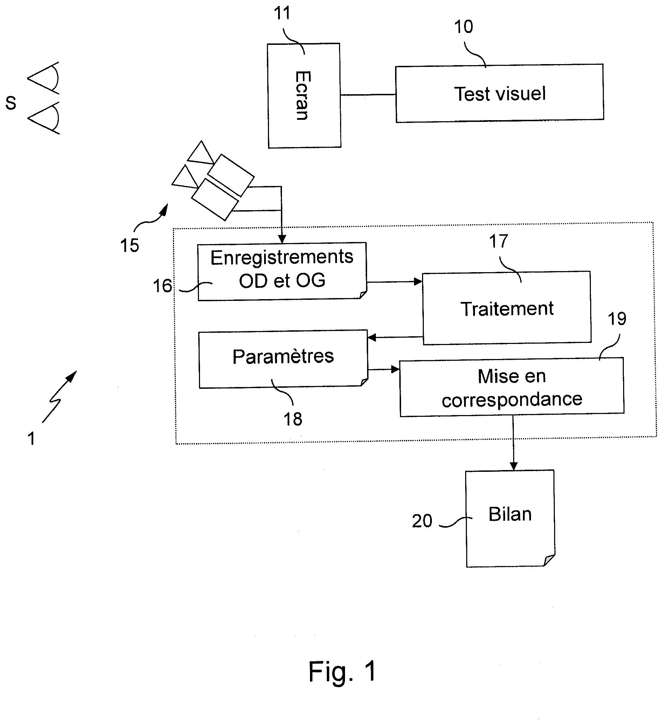

Dans le mode de réalisation de la **figure 1****,** un système 1 d'aide à la détection d'une anormalité oculomotrice comprend une partie de test et une partie d'acquisition et de traitement.

La partie de test comprend notamment un module 10 de test pour soumettre le sujet S à un test visuel impliquant au moins un retour à la ligne d'au moins un œil du sujet, incluant une tâche de lecture d'une suite de symboles répartie sur plusieurs lignes horizontales et/ou une tâche de recherche visuelle d'occurrences d'un symbole au sein de la suite de symboles, ainsi qu'un écran 11 pour afficher cette suite de symboles au sujet à une distance comprise entre environ 45 et 65 cm, de préférence environ 60 cm.

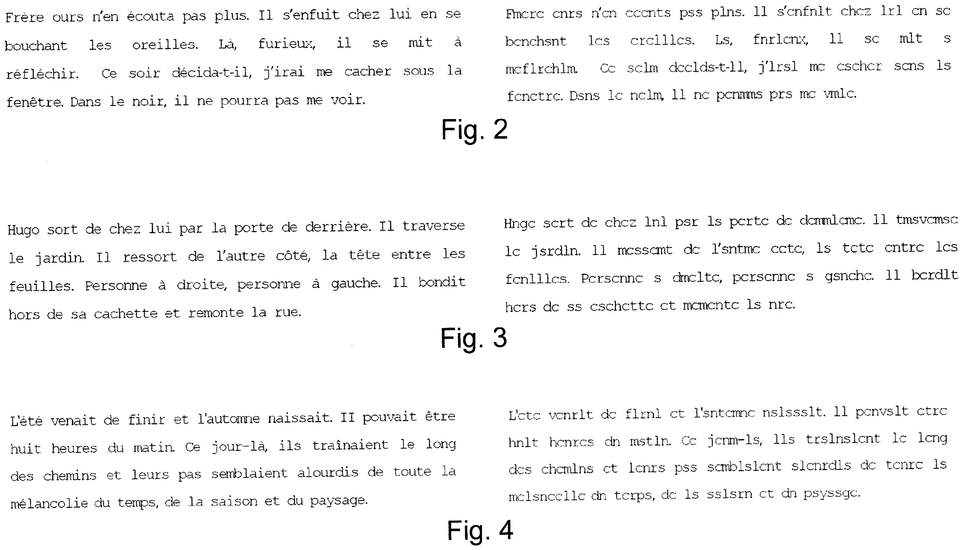

Les **figures 2 à 4** illustrent des exemples de paragraphes présentés au sujet pour le test visuel.

Les paragraphes dotés d'un contenu sémantique sur la gauche de ces figures sont dédiés à la tâche de lecture, alors que les paragraphes situés sur la droite de ces figures sont dédiés à la tâche de recherche visuelle en incorporant uniquement des consonnes sans contenu sémantique.

Dans les expériences menées et présentées par la suite, un paragraphe sélectionné en fonction du test à conduire et de l'âge du sujet S est affiché dans un champ de vision du sujet S avec une largeur comprise entre 20 et 40 degrés, de préférence entre 25 et 30 degrés, avantageusement de 29 degrés et une hauteur comprise entre 5 et 10 degrés, avantageusement de 6,4 degrés.

Les textes présentés au sujet comprennent avantageusement plusieurs lignes, par exemple entre 2 et 10 lignes, notamment entre 3 et 5 lignes.

Dans l'exemple des figures, ces textes comprennent 4 lignes, formées ensemble d'environ 40 mots et de 180 caractères. Ainsi les yeux du sujet sont amenés à réaliser trois retours à la ligne. En pratique, on prévoit des tests impliquant entre 1 et 10 retours à la ligne, préférablement entre 2 et 6 retours à la ligne, par exemple 3 ou plus, notamment 4 ou 5.

Chaque ligne peut comprendre entre 30 et 100 symboles (ici des caractères typographiques) de sorte à former entre 7 et 20 mots. Ainsi le sujet est amené à réaliser plusieurs saccades progressives horizontales pour parcourir ou balayer la ligne et des saccades de retour pour passer d'une ligne à l'autre.

Le module de test 10 donne pour instruction au sujet S, soit de lire le texte pour la tâche de lecture, soit de rechercher une lettre, par exemple la lettre "r", dans le paragraphe affiché pour la tâche de recherche visuelle.

Le sujet peut être amené à réaliser successivement les deux tâches.

De retour à la **figure 1****,** la partie d'acquisition et de traitement comprend un module d'acquisition vidéo 15 placé en face des yeux du sujet pour acquérir à forte cadence (par exemple toutes les 4 ms) le déplacement de chacun des yeux pendant le test visuel.

Le module d'acquisition vidéo 15 réalise un prétraitement des images acquises, selon des techniques classiques de traitement de l'image, pour obtenir un enregistrement, dans le temps, du déplacement de chaque œil en amplitude selon l'axe horizontal (degrés par rapport au centre de vision).

Le module d'acquisition vidéo 15 réalise le suivi des mouvements oculaires pour les deux yeux simultanément. A cet effet, le système Mobile Eyebrain Tracker (Mobile EBT®) peut être utilisé.

Les deux courbes obtenues 16 sont alors enregistrées dans le système pour un traitement immédiat ou ultérieur au recueil des données.

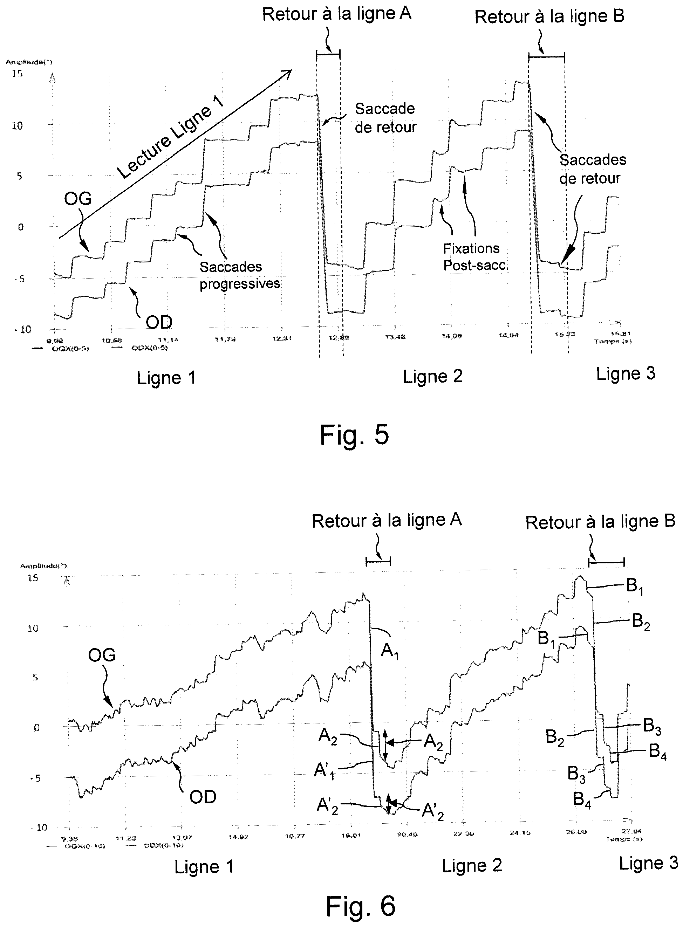

La **figure 5** représente une portion des enregistrements pour l'œil droit OD (courbe du bas) et pour l'œil gauche OG (courbe du haut), chez un sujet non atteint de dyslexie, lors d'un test de lecture (**figure 2, 3** ou **4**). Sont représentés les enregistrements lors de la lecture de deux lignes et du début d'une troisième ligne.

La partie d'acquisition et de traitement du système 1 comprend également un module de traitement numérique 17 configuré pour déterminer, dans les enregistrements 16, des saccades de retour composant tout ou partie d'au moins un retour à la ligne de l'œil du sujet et pour calculer au moins un paramètre 18 de saccade de retour à partir des enregistrements correspondant aux saccades de retour déterminées.

Ce module 17 réalise notamment l'identification de saccades et de fixations post-saccadiques sur les enregistrements 16, et notamment l'identification de saccades composant des retours à la ligne.

Comme décrit dans la publication *C. Prado et al.* une saccade peut être identifiée aux endroits où les mouvements de l'œil présentent un pic de vitesse supérieur à 30°/s et inférieur à 800°/s et une amplitude de déplacement supérieure à 0,5 ou 1 degré par rapport à la position de fixation avant le déclenchement de la saccade. Le début et la fin de la saccade peuvent être délimités par le changement de signe de la vitesse sur deux points arrière. Bien entendu d'autres critères peuvent être mis en œuvre, notamment, par exemple, un algorithme où le seuil de détection de la vitesse est dynamique, c'est-à-dire moyenné par sujet en général pour une durée de signal inférieur à 20 secondes. La vitesse est alors calculée, de préférence, en 3 points centrés.

Sur l'exemple de la **figure 5****,** des saccades sont ainsi identifiées entre les instants 9,98s et 12,31s correspondant à des saccades progressives effectuées lors de la lecture d'une première ligne. De même les saccades entre les instants 12,98s et 14.80s correspondent à des saccades progressives effectuées lors de la lecture d'une deuxième ligne. On observe que chaque saccade représente un déplacement significatif des yeux d'environ +1 à +5 degrés (zone de transition entre deux paliers horizontaux). La détection des saccades progressives et de fixations post-saccadiques (périodes entre deux saccades, c'est-à-dire les paliers horizontaux sur la figure) n'est pas nécessaire aux fins de la présente invention.

Des saccades de retour sont également identifiées dans l'enregistrement. L'identification de ces saccades par rapport aux saccades progressives peut reposer sur le fait qu'elles présentent une amplitude inverse (ici négative). En outre pour les identifier par rapport à des saccades régressives (non représentées) effectuées lors de la lecture de la ligne, un retour à la ligne est caractérisé par une première saccade régressive non suivie par une période de fixation relativement longue (une fixation est définie par un plateau sur le tracé. Elles sont, dans la lecture, d'une durée variant de 100 à 600 ms), cette première saccade se situant en bout de ligne.

La première saccade peut être de forte amplitude en valeur absolue (ici -17 degrés à comparer aux +1 à +5 des saccades en lecture de ligne). Ainsi un seuil par exemple fixé à 10 degrés pour un texte présenté sur 29 degrés (soit un ratio d'1/3) pourrait permettre de détecter une première saccade de retour, dans un mode de réalisation.

Les saccades d'amplitude négative suivant cette première saccade sont également des saccades de retour constitutives du même retour à la ligne, jusqu'à ce que les yeux du sujet atteignent la position angulaire correspondant à un début de ligne (-9 degrés environ sur la figure).

Toutes les saccades successives ainsi mesurées, non suivies d'une période de fixation relativement longue (supérieur à 100 ms par exemple) et/ou précédant une saccade progressive de lecture, sont considérées comme faisant partie du même retour à la ligne.

Dans l'exemple représenté sur la figure, le premier retour à la ligne est constitué d'une seule saccade de retour, et le deuxième retour à la ligne est constitué de deux saccades de retour.

Une fois les retours à la ligne et leur saccades de retour identifiés, le module de traitement 17 détermine et calcule notamment un ou plusieurs paramètres de saccade de retour comme décrit par la suite. Eventuellement d'autres paramètres classiques peuvent également être calculés pour être utilisés en combinaison avec ce ou ces paramètres de saccade de retour dans le cadre de la détection de troubles oculomoteurs.

Enfin, la partie d'acquisition et de traitement du système 1 comprend un module 19 pour mettre en correspondance la ou les valeurs déterminées pour les paramètres de saccade de retour 18 avec au moins une ou des valeurs seuils VS correspondantes de sorte à déterminer une anormalité oculomotrice chez le sujet testé.

Ce module peut consister par exemple à émettre un bilan oculomoteur 20 sur lequel apparaissent le ou les divers paramètres déterminés par le module 17, ainsi que la ou les valeurs seuils correspondantes.

En variante, ce module peut opérer une comparaison numérique entre la ou les valeurs des paramètres considérés et leur valeur seuil correspondante pour émettre une information décisionnelle du type "sujet dyslexique" ou "sujet non-dyslexique".

La **figure 6** représente une portion des enregistrements pour l'œil droit OD (courbe du bas) et pour l'œil gauche OG (courbe du haut) chez un sujet atteint de dyslexie, lors du même test de lecture (**figure 2, 3** ou **4**). Sur la figure sont représentés les enregistrements lors de la lecture des trois lignes. Deux retours à la ligne, notés A et B, sont donc identifiés par le module 17 à l'intérieur de cet enregistrement.

Le module 17 détermine notamment tout ou partie des paramètres décrits ci-après pour une pluralité de retours à la ligne. Ces mêmes paramètres peuvent être déterminés lorsqu'un seul retour à la ligne est disponible dans les enregistrements 16.

Certains paramètres sont calculés pour un œil donné (paramètres monoculaires). On peut donc procéder à un calcul similaire pour l'autre œil afin de disposer d'un plus grand nombre d'informations. Pour la suite de la description, on fait référence à un seul œil, par exemple OG. Bien entendu, les enseignements qui suivent peuvent donc être appliqués pour l'autre œil en variante ou en combinaison.

Ces paramètres monoculaires reposant sur l'enregistrement 16 d'un seul œil comprennent :
- un nombre moyen de saccades de retour sur une pluralité de retours à la ligne lors de la tâche de lecture,
- un moyenne de ratios sur la pluralité de retours à la ligne lors de la tâche de lecture, chaque ratio pour un retour à ligne correspondant au ratio entre l'amplitude horizontale de la première saccade temporelle de retour dans ledit retour à la ligne et l'amplitude totale horizontale des saccades de retour constitutives du même retour à la ligne,

Les mêmes paramètres peuvent être obtenus lorsque le sujet est soumis au test de recherche visuelle (textes de droite des **figures 2, 3** et **4**) :
- un nombre moyen de saccades de retour sur une pluralité de retours à la ligne lors de la tâche de recherche visuelle,
- un moyenne de ratios sur la pluralité de retours à la ligne lors de la tâche de recherche visuelle, chaque ratio pour un retour à ligne correspondant au ratio entre l'amplitude horizontale de la première saccade temporelle de retour dans ledit retour à la ligne et l'amplitude totale horizontale des saccades de retour constitutives du même retour à la ligne,

Dans l'exemple de la **figure 6****,** le module 17 a identifié deux saccades de retour, notées A1 et A2, constitutives du premier retour à la ligne A, et identifié quatre saccades de retour, notées B1, B2, B3 et B4, constitutives du deuxième retour à la ligne B.

Le nombre N_{A} de saccades de retour pour le premier retour à la ligne A est donc 2, et le nombre N_{B} de saccades de retour pour le deuxième retour à la ligne B est 4. D'autres retours à la ligne L (non représentés) peuvent également avoir leur propre nombre N_{L} de saccades de retour.

Le nombre moyen N_{moy} de saccades de retour sur une pluralité de retours à la ligne lors du test visuel (lecture ou recherche visuelle) est ainsi la moyenne de ces nombres N_{L} de saccades de retour, à savoir N_{moy} = (∑N_{L}) / (nombre de retours à la ligne).

Dans l'exemple de la figure, N_{moy} = (N_{A} + N_{B})/2 = 3.

Le module 17 détermine également l'amplitude horizontale de chaque saccade de retour Aᵢ, Bⱼ, etc. Pour simplifier les explications, on note A₁ l'amplitude horizontale de la saccade de retour A₁, A₂ celle de la saccade de retour A₂, etc. Ces amplitudes horizontales sont exprimées en degrés et correspondent au déplacement de l'œil lors de la saccade de retour (déplacement vertical sur les enregistrements de la figure).

Puis le module 17 calcule le gain G_{L} de chaque retour à la ligne L. Le gain G_{L} est le ratio entre l'amplitude horizontale de la première saccade temporelle de retour L₁ et l'amplitude horizontale totale du retour à la ligne, par exemple la somme des amplitudes horizontales des saccades de retour constitutives du retour à la ligne.

Dans une variante, l'amplitude horizontale totale peut être calculée comme l'amplitude entre les deux positions extrêmes du même œil (c'est-à-dire les deux points les plus haut et les plus bas sur la courbe OG de la figure).

Dans l'exemple de la figure, G_{A} = A₁ / (A₁ + A₂) et G_{B} = B₁ / (B₁ + B₂ + B₃ + B₄)

La moyenne G_{moy} des ratios (ou gains) sur la pluralité de retours à la ligne lors du test visuel est ainsi la moyenne des gains G_{L} calculés pour l'ensemble des retours à la ligne considérés : G_{moy} = (∑G_{L}) / (nombre de retours à la ligne). Dans l'exemple de la figure, G_{moy} = (G_{A} + G_{B}) / 2.

Outre ces paramètres de saccade de retour monoculaires, un paramètre binoculaire est également calculé par le module 17. Ce paramètre binoculaire est un paramètre de disconjugaison binoculaire en amplitude représentatif d'une différence de mouvement horizontal entre les deux yeux du sujet lors d'au moins une saccade de retour déterminée.

Notons A'₁ l'amplitude horizontale de la première saccade temporelle de retour pour l'œil droit OD lors du retour à la ligne A (A₁ est l'amplitude pour l'OG pour la même première saccade temporelle de retour), A'₂ celle de la deuxième saccade de retour pour OD lors du même retour à la ligne A, etc.

A noter qu'il est possible qu'un œil fasse plus de saccades de retour que le deuxième. Il a été observé cependant que ces saccades de retour supplémentaires sont généralement de très faibles amplitudes comparées aux saccades de retour principales du retour à la ligne. Ainsi, dans un mode de réalisation, il est prévu de détecter/identifier uniquement les saccades de retour qui présentent une amplitude supérieure à 1 °. Par l'application de cette amplitude seuil, les deux yeux présentent le même nombre de saccades de retour dans la plupart des situations.

Toutefois une deuxième valeur seuil est appliquée dans les autres situations pour mesurer le paramètre de disconjugaison binoculaire. Cette valeur seuil correspond à un seuil temporel de 80 ms, au-delà duquel si une nouvelle saccade de retour a été détectée sur un œil mais pas sur l'autre, alors la saccade de retour est comptabilisée comme étant une saccade de retour mais aucun paramètre de disconjugaison binoculaire n'est calculé sur cette portion particulière d'enregistrement des mouvements des yeux. Une fois cette nouvelle saccade de retour traitée uniquement de façon monoculaire, la détection d'une autre saccade de retour est resynchronisée sur les deux yeux.

Le paramètre δ de disconjugaison binoculaire pour une saccade de retour peut être calculé par le module 17 comme un ratio entre la différence d'amplitude horizontale entre OD et OG lors de la même saccade de retour et l'amplitude horizontale de cette saccade, à savoir δ_{A2} = (A₂ - A'₂) / A₂ * 100.

Dans cet exemple, l'amplitude A₂ est utilisée comme valeur de l'amplitude horizontale de la saccade. En variante, on peut prendre A'₂, ou faire une moyenne des deux amplitudes.

Chaque paramètre δ_{Li} de disconjugaison binoculaire pour une saccade de retour Lᵢ peut être utilisé indépendamment des autres dans le processus de détection d'un trouble oculomoteur. En variante, un paramètre moyen δ_{L} de disconjugaison binoculaire pour l'ensemble d'un retour à la ligne L peut être calculé : δ_{L}= (Σδ_{Li}) / N_{L}, où N_{L} est le nombre de saccades de retour constitutives du retour à la ligne L. Dans une autre variante, un paramètre global Δ de disconjugaison binoculaire pour l'ensemble des retours à la ligne peut être calculé : Δ= (∑δ_{L}) / (nombre de retours à la ligne).

On décrit maintenant les résultats de tests de recherche visuelle réalisés sur 30 enfants dyslexiques et 42 enfants normo-lecteurs âgés de 8 à 13 ans, testés en fonction de leur âge (**figures 5** à **11b**).

Les enfants normo-lecteurs ont notamment permis d'établir des normales et des valeurs seuils correspondant à p<.05 (c'est-à-dire 5% équivalent à deux fois la déviation standard à la moyenne) pour tout ou partie des paramètres mentionnés précédemment. L'invention n'est cependant pas limitée à ce type de seuillage, d'autres techniques pouvant être mises en œuvre pour améliorer ou relâcher des contraintes sur la présence de faux-positifs.

Les enfants sont répartis en trois classes d'âges : 8-9 ans ; 10-11 ans et 12-13 ans.

Pour les enfants de la classe 8-9 ans (15 enfants normo-lecteurs et 14 enfants dyslexiques), le test visuel est effectué sur l'un des paragraphes de la **figure 2****.** Pour ceux âgés de 10-11 ans (16 enfants normo-lecteurs et 11 enfants dyslexiques), le test visuel est effectué sur l'un des paragraphes de la **figure 3****.** Enfin pour ceux âgés de 12-13 ans (11 enfants normo-lecteurs et 5 enfants dyslexiques), le test visuel est effectué sur l'un des paragraphes de la **figure 4****.**

Les mouvements oculaires ont été enregistrés par le système de vidéo-oculographie Mobile EBT®.

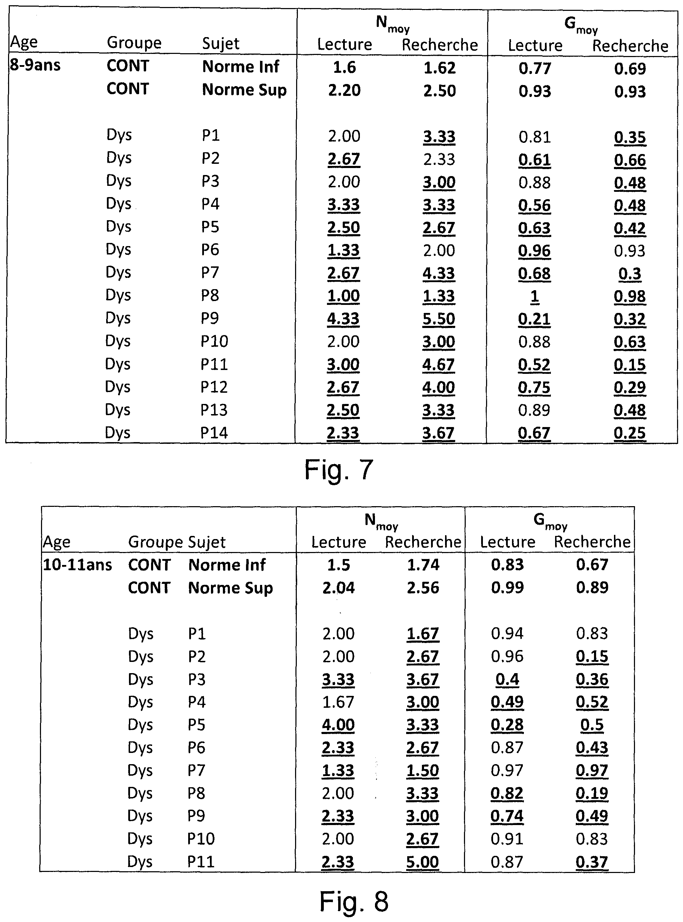

Les tableaux des **figures 7, 8** et **9** illustre les résultats obtenus pour les trois classes d'âges à partir des enregistrements de OD et OG. Les valeurs inscrites dans ces tableaux sont en effet des moyennes pour OD et OG. Bien entendu, des tableaux similaires peuvent être établis pour OD uniquement et/ou pour OG uniquement.

Les enfants normo-lecteurs (sujets sains non dyslexiques de chaque groupe d'âge) servent de contrôles pour établir les normales des paramètres susmentionnés, ainsi que les valeurs seuils correspondantes (p<.05), en particulier des bornes inférieure et supérieure comme reprises dans les tableaux.

Ces bornes sont comparées aux paramètres de saccade de retour respectivement calculés afin d'identifier une éventuelle anomalie oculomotrice.

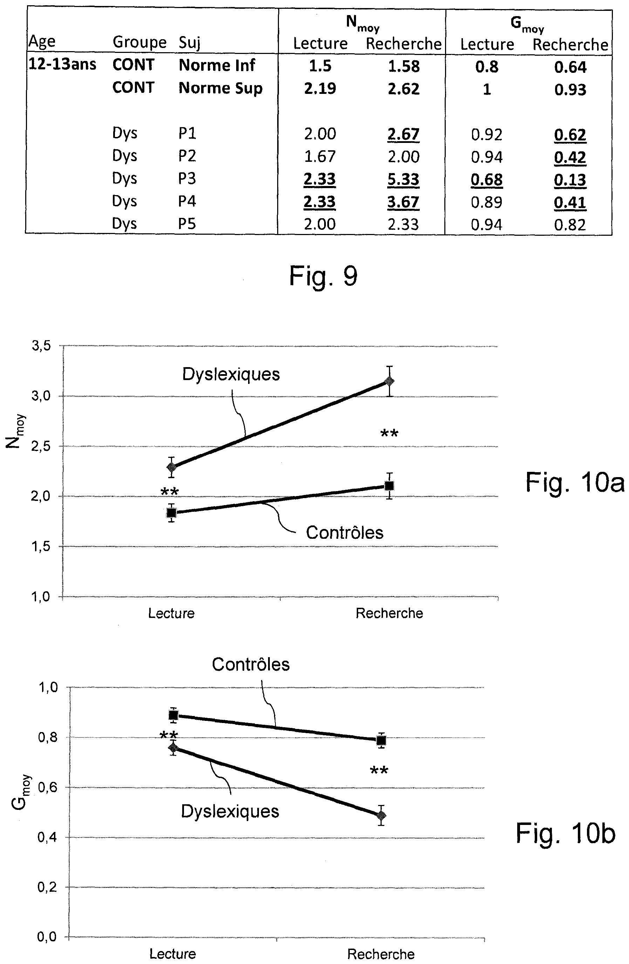

Ces résultats montrent :
- que les enfants dyslexiques font plus de saccades de retour à la ligne (N_{moy}) que les enfants sains (F(1,66)=25.75, p<0.001), comme schématiquement montré sur la **figure 10a****.** Ceci est vrai dans un test de lecture (p<0.008) et dans un test de recherche visuelle (p<0.001). On prévoit ainsi que la comparaison effectuée par le module 19 détermine préférentiellement si un paramètre sur le nombre moyen de saccades de retour dépasse la borne supérieure obtenue des sujets sains, ou éventuellement la borne inférieure ;
- que les enfants dyslexiques ont un gain G_{moy} plus faible que les enfants sains (F(1,66)=31.39, p<0.0001), comme schématiquement montré sur la **figure 10b****.** Cela signifie que les enfants dyslexiques ont une première saccade temporelle de retour qui est moins précise que les sujets sains. Ceci est vrai dans un test de lecture (p<0.007) et dans un test de recherche (p<0.001). On prévoit ainsi que la comparaison effectuée par le module 19 détermine préférentiellement si un paramètre sur le gain moyen des saccades de retour dépasse la borne inférieure obtenue des sujets sains, ou éventuellement la borne supérieure.

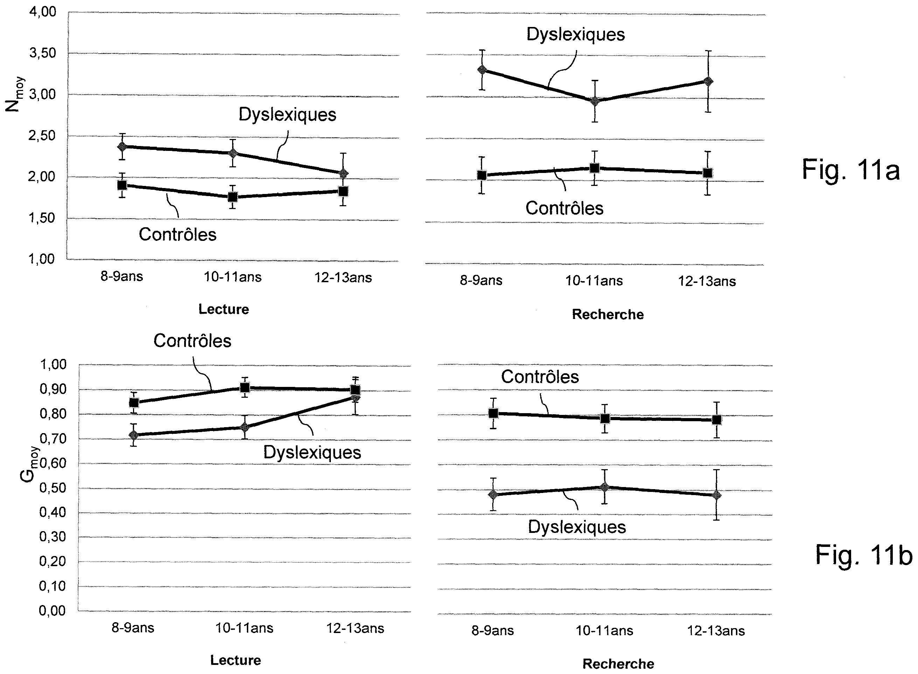

Ces résultats ne sont pas modulés par l'âge, ni sur le nombre de saccades (**figure 11a****,** F(2,62)=1.37, p=0.27), ni sur le gain (**figure 11b****,** F<1)
Le tableau de la **figure 7** montre notamment que la totalité des enfants dyslexiques de la tranche d'âges 8-9 ans ont au moins un paramètre monoculaire de retour à la ligne moyenné pour OD et OG (sur les quatre définis plus haut) qui est déficitaire (les valeurs en gras souligné sont hors bornes). On notera que la majorité des enfants dyslexiques a au moins soit un paramètre N_{moy} supérieur à la borne supérieure de référence, soit un paramètre G_{moy} inférieur à la borne inférieure de référence. Toutefois deux exceptions, dyslexiques, dépassent les autres bornes de référence (P6 et P8).

Le tableau de la **figure 8** montre notamment que la totalité des enfants dyslexiques de la tranche d'âges 10-11 ans ont au moins un paramètre monoculaire de retour à la ligne moyenné (sur les quatre définis plus haut) qui est déficitaire (valeurs en gras souligné). On notera que la majorité des enfants dyslexiques a au moins soit un paramètre N_{moy} supérieur à la borne supérieure de référence, soit un paramètre G_{moy} inférieur à la borne inférieure de référence. Deux exceptions, dyslexiques, ont au moins un de leurs paramètres qui dépassent les autres bornes de référence (P1 et P7).

Le tableau de la **figure 9** montre notamment que 80% des enfants dyslexiques testés dans la tranche d'âges 12-13 ans ont au moins un paramètre monoculaire de retour à la ligne moyenné (sur les quatre définis plus haut) qui est déficitaire (valeurs en gras souligné).

La combinaison des quatre paramètres monoculaires moyennés (N_{moy} et G_{moy} pour chacun des deux tests) permet ainsi de détecter assez précisément les sujets dyslexiques.

Le paramètre binoculaire de saccade de retour (le paramètre de disconjugaison binoculaire en amplitude défini plus haut) peut également être utilisé dans la détection d'un trouble oculomoteur par le module 19.

Les résultats obtenus sur les mêmes groupes d'enfants montrent que ce paramètre de disconjugaison binoculaire en amplitude est substantiellement plus important chez les sujets dyslexiques que chez les sujets sains.

Notamment les résultats obtenus montrent un paramètre moyen de disconjugaison calculé sur l'ensemble des saccades de retour d'un même retour à la ligne, lequel paramètre moyen de disconjugaison est égal à 7,4 % chez les sujets sains en tâche de lecture et égal à 8,9 % chez les mêmes sujets sains en tâche de recherche visuelle.

En comparaison, ce paramètre moyen de disconjugaison est de 13,8 % chez les sujets dyslexiques en tâche de lecture et de 13,3 % chez les mêmes sujets dyslexiques en tâche de recherche visuelle.

Par conséquent une valeur seuil pour la comparaison par le module 19 afin d'identifier un trouble oculomoteur à l'aide de ce paramètre de disconjugaison binoculaire en amplitude peut être fixé à une valeur comprise entre 6 % et 11 %, par exemple 8 %. Ainsi, une anormalité oculomotrice est détectée dès lors que ce paramètre de disconjugaison binoculaire en amplitude est supérieur à cette valeur seuil.

La présente invention permet de collecter et obtenir un ou plusieurs paramètres particuliers relatifs à des saccades de retour formant des retours à la ligne, qui s'avèrent efficaces dans la détection et l'identification de sujets atteints d'une anormalité oculomotrice par un simple test visuel. En particulier, les résultats obtenus à partir de ces paramètres montrent que les sujets dyslexiques présentent un nouveau type de trouble oculomoteur caractérisé par une modification substantielle du nombre de saccades de retour ou du gain moyen lors des retours à la ligne, ainsi que par la présence d'une disconjugaison binoculaire lors de ces saccades.

Les exemples qui précèdent ne sont que des modes de réalisation de l'invention qui ne s'y limite pas.

## Revendications

1. Procédé comprenant les étapes suivantes :
soumettre le sujet (S) à un test visuel impliquant au moins un retour à la ligne (A, B, L) d'au moins un œil du sujet;
pendant ladite tâche, enregistrer les mouvements de l'œil du sujet à l'aide d'un dispositif de suivi de mouvements oculaires (15);
déterminer, à l'aide d'un dispositif de traitement numérique (17) et dans les enregistrements obtenus (16), des saccades de retour (A₁, A₂, B₁-B₄, Lᵢ) composant tout ou partie de l'au moins un retour à la ligne de l'œil du sujet;
calculer au moins un paramètre de saccade de retour (18, N_{moy}, G_{moy}, Δ) à partir des enregistrements correspondant aux saccades de retour déterminées, procédé dans lequel l'au moins un paramètre de saccade de retour est fonction d'un nombre de saccades de retour (N_{A}, N_{B}, N_{L}) constitutives d'un même retour à la ligne (A, B, L) et comprend un nombre moyen de saccades de retour (N_{moy}) sur une pluralité de retours à la ligne.

2. Procédé comprenant les étapes suivantes :
soumettre le sujet (S) à un test visuel impliquant au moins un retour à la ligne (A, B, L) d'au moins un œil du sujet;
pendant ladite tâche, enregistrer les mouvements de l'œil du sujet à l'aide d'un dispositif de suivi de mouvements oculaires (15);
déterminer, à l'aide d'un dispositif de traitement numérique (17) et dans les enregistrements obtenus (16), des saccades de retour (A₁, A₂, B₁-B₄, Lᵢ) composant tout ou partie de l'au moins un retour à la ligne de l'œil du sujet;
calculer au moins un paramètre de saccade de retour (18, N_{moy}, G_{moy}, Δ) à partir des enregistrements correspondant aux saccades de retour déterminées, procédé dans lequel l'au moins un paramètre de saccade de retour est fonction d'un ratio (G_{A}, G_{B}, G_{L}) entre une amplitude horizontale d'une saccade de retour donnée dans un retour à la ligne et une amplitude totale horizontale de saccades de retour constitutives du même retour à la ligne.

3. Procédé selon la revendication 1, dans lequel la saccade de retour donnée est la première saccade temporelle (A₁, B₁, L₁) à l'intérieur du retour à la ligne.

4. Procédé selon la revendication 1 ou 3, dans lequel l'au moins un paramètre de saccade de retour comprend une moyenne (G_{moy}) de ratios sur une pluralité de retours à la ligne, chaque ratio (G_{A}, G_{B}, G_{L}) pour un retour à ligne correspondant au ratio entre une amplitude horizontale de la première saccade temporelle de retour dans ledit retour à la ligne et une amplitude totale horizontale de saccades de retour constitutives du même retour à la ligne.

5. Procédé comprenant les étapes suivantes :
soumettre le sujet (S) à un test visuel impliquant au moins un retour à la ligne (A, B, L) d'au moins un œil du sujet;
pendant ladite tâche, enregistrer les mouvements de l'œil du sujet à l'aide d'un dispositif de suivi de mouvements oculaires (15);
déterminer, à l'aide d'un dispositif de traitement numérique (17) et dans les enregistrements obtenus (16), des saccades de retour (A₁, A₂, B₁-B₄, Lᵢ) composant tout ou partie de l'au moins un retour à la ligne de l'œil du sujet;
calculer au moins un paramètre de saccade de retour (18, N_{moy}, G_{moy}, Δ) à partir des enregistrements correspondant aux saccades de retour déterminées, procédé dans lequel l'au moins un paramètre de saccade de retour combine un nombre moyen de saccades de retour (N_{moy}) sur une pluralité de retours à la ligne et une moyenne (G_{moy}) de ratios sur la pluralité de retours à la ligne, chaque ratio pour un retour à ligne correspondant au ratio entre une amplitude horizontale de la première saccade temporelle de retour dans ledit retour à la ligne et une amplitude totale horizontale de saccades de retour constitutives du même retour à la ligne.

6. Procédé selon la revendication 5, le procédé comportant l'étape consistant à comparer le nombre moyen de saccades de retour à une première valeur seuil (VS) ou la moyenne de ratios à une deuxième valeur seuil (VS).

7. Procédé comprenant les étapes suivantes :
soumettre le sujet (S) à un test visuel impliquant au moins un retour à la ligne (A, B, L) d'au moins un œil du sujet;
pendant ladite tâche, enregistrer les mouvements de l'œil du sujet à l'aide d'un dispositif de suivi de mouvements oculaires (15);
déterminer, à l'aide d'un dispositif de traitement numérique (17) et dans les enregistrements obtenus (16), des saccades de retour (A₁, A₂, B₁-B₄, Lᵢ) composant tout ou partie de l'au moins un retour à la ligne de l'œil du sujet;
calculer au moins un paramètre de saccade de retour (18, N_{moy}, G_{moy}, Δ) à partir des enregistrements correspondant aux saccades de retour déterminées, procédé dans lequel les mouvements de deux yeux du sujet sont enregistrés dans lesquels des saccades de retour sont déterminées, et le paramètre de saccade de retour comprend au moins un paramètre de disconjugaison binoculaire en amplitude (δ_{A2}, δ_{L}, Δ) représentatif d'une différence de mouvement horizontal entre les deux yeux du sujet lors d'au moins une saccade de retour déterminée.

8. Procédé selon la revendication 1, dans lequel le test visuel comprend une tâche de lecture d'une suite de symboles répartie sur plusieurs lignes horizontales ou une tâche de recherche visuelle d'occurrences d'un symbole au sein de la suite de symboles.

9. Procédé selon la revendication 1, comprenant la comparaison du paramètre calculé avec au moins une valeur seuil de référence.

10. Procédé comprenant les étapes suivantes :
soumettre le sujet (S) à un test visuel impliquant au moins un retour à la ligne (A, B, L) d'au moins un œil du sujet;
pendant ladite tâche, enregistrer les mouvements de l'œil du sujet à l'aide d'un dispositif de suivi de mouvements oculaires (15);
déterminer, à l'aide d'un dispositif de traitement numérique (17) et dans les enregistrements obtenus (16), des saccades de retour (A₁, A₂, B₁-B₄, Lᵢ) composant tout ou partie de l'au moins un retour à la ligne de l'œil du sujet;
calculer au moins un paramètre de saccade de retour (18, N_{moy}, G_{moy}, Δ) à partir des enregistrements correspondant aux saccades de retour déterminées, procédé dans lequel on soumet le sujet (S) à une tâche de lecture d'une suite de symboles répartie sur plusieurs lignes horizontales et à une tâche de recherche visuelle d'occurrences d'un symbole au sein d'une suite de symboles répartie sur plusieurs lignes horizontales; et
le procédé comprenant comparer au moins un paramètre (18) parmi
un nombre moyen de saccades de retour (N_{moy}) sur une pluralité de retours à la ligne lors de la tâche de lecture,
un moyenne (G_{moy}) de ratios sur la pluralité de retours à la ligne lors de la tâche de lecture, chaque ratio pour un retour à ligne correspondant au ratio entre une amplitude horizontale de la première saccade temporelle de retour dans ledit retour à la ligne et une amplitude totale horizontale des saccades de retour constitutives du même retour à la ligne,
un nombre moyen de saccades de retour (N_{moy}) sur une pluralité de retours à la ligne lors de la tâche de recherche visuelle,
une moyenne (G_{moy}) de ratios sur la pluralité de retours à la ligne lors de la tâche de recherche visuelle, chaque ratio pour un retour à ligne correspondant au ratio entre une amplitude horizontale de la première saccade temporelle de retour dans ledit retour à la ligne et une amplitude totale horizontale des saccades de retour constitutives du même retour à la ligne,
à une valeur seuil correspondante (VS).

11. Système (1) comprenant:
- un module de test (10) pour soumettre le sujet (S) à un test visuel impliquant au moins un retour à la ligne (A, B, L) d'au moins un œil du sujet;
- un module (15) de suivi de mouvements oculaires configuré pour enregistrer, pendant ladite tâche, les mouvements d'au moins un œil du sujet;
- un module (17) de traitement numérique configuré pour déterminer, dans les enregistrements obtenus (16), des saccades de retour (A₁, A₂, B₁-B₄, Lᵢ) composant tout ou partie de l'au moins un retour à la ligne de l'œil du sujet;
- un module pour calculer au moins un paramètre de saccade de retour (18, N_{moy}, G_{moy}, Δ), où l'au moins un paramètre de saccade de retour est fonction d'un nombre de saccades de retour (N_{A}, N_{B}, N_{L}) constitutives d'un même retour à la ligne (A, B, L) et comprend un nombre moyen de saccades de retour (N_{moy}) sur une pluralité de retours à la ligne, à partir des enregistrements correspondant aux saccades de retour déterminées.

## Patentansprüche

1. Verfahren, umfassend die folgenden Schritte:
die Person (S) einem Sehtest unterziehen, der mindestens einen Zeilenrücksprung (A, B, L) mindestens eines Auges der Person beinhaltet;
während der Aufgabe Aufzeichnen der Bewegungen des Auges der Person mithilfe einer Vorrichtung zur Verfolgung von Augenbewegungen (15);
Bestimmen, mithilfe einer digitalen Verarbeitungsvorrichtung (17) und in den erhaltenen Aufzeichnungen (16), der Rückwärtssakkaden (A₁, A₂, B₁-B₄, Lᵢ), aus denen sich der gesamte oder ein Teil des mindestens einen Zeilenrücksprungs des Auges der Person zusammensetzt;
Berechnen mindestens eines Rückwärtssakkadenparameters (18, N_{moy}, G_{moy}, Δ) anhand der Aufzeichnungen, die den bestimmten Rückwärtssakkaden entsprechen, wobei bei dem Verfahren mindestens ein Rückwärtssakkadenparameter von einer Anzahl von Rückwärtssakkaden (N_{A}, N_{B}, N_{L}) abhängig ist, aus denen sich ein und derselbe Zeilenrücksprung (A, B, L) zusammensetzt, und eine mittlere Anzahl von Rückwärtssakkaden (N_{moy}) über eine Vielzahl von Zeilenrücksprüngen umfasst.

2. Verfahren, umfassend die folgenden Schritte:
die Person (S) einem Sehtest unterziehen, der mindestens einen Zeilenrücksprung (A, B, L) mindestens eines Auges der Person beinhaltet;
während der Aufgabe Aufzeichnen der Bewegungen des Auges der Person mithilfe einer Vorrichtung zur Verfolgung von Augenbewegungen (15);
Bestimmen, mithilfe einer digitalen Verarbeitungsvorrichtung (17) und in den erhaltenen Aufzeichnungen (16), der Rückwärtssakkaden (A₁, A₂, B₁-B₄, Lᵢ), aus denen sich der gesamte oder ein Teil des mindestens einen Zeilenrücksprungs des Auges der Person zusammensetzt;
Berechnen mindestens eines Rückwärtssakkadenparameters (18, N_{moy}, G_{moy}, Δ) anhand der Aufzeichnungen, die den bestimmten Rückwärtssakkaden entsprechen, wobei bei dem Verfahren der mindestens eine Rückwärtssakkadenparameter von einem Verhältnis (G_{A}, G_{B}, G_{L}) zwischen einer horizontalen Amplitude einer gegebenen Rückwärtssakkade in einem Zeilenrücksprung und einer horizontalen Gesamtamplitude von Rückwärtssakkaden, aus denen sich ein und derselbe Zeilenrücksprung zusammensetzt, abhängig ist.

3. Verfahren nach Anspruch 1, bei dem die gegebene Rückwärtssakkade die zeitlich erste Sakkade (A₁, B₁, L₁) innerhalb des Zeilenrücksprungs ist.

4. Verfahren nach Anspruch 1 oder 3, bei dem der mindestens eine Rückwärtssakkadenparameter einen Mittelwert (G_{moy}) von Verhältnissen über eine Vielzahl von Zeilenrücksprüngen umfasst, wobei jedes Verhältnis (G_{A}, G_{B}, G_{L}) für einen Zeilenrücksprung dem Verhältnis zwischen einer horizontalen Amplitude der zeitlich ersten Rückwärtssakkade in dem Zeilenrücksprung und einer horizontalen Gesamtamplitude von Rückwärtssakkaden, aus denen sich ein und derselbe Zeilenrücksprung zusammensetzt, entspricht.

5. Verfahren, umfassend die folgenden Schritte:
die Person (S) einem Sehtest unterziehen, der mindestens einen Zeilenrücksprung (A, B, L) mindestens eines Auges der Person beinhaltet;
während der Aufgabe Aufzeichnen der Bewegungen des Auges der Person mithilfe einer Vorrichtung zur Verfolgung von Augenbewegungen (15);
Bestimmen, mithilfe einer digitalen Verarbeitungsvorrichtung (17) und in den erhaltenen Aufzeichnungen (16), der Rückwärtssakkaden (A₁, A₂, B₁-B₄, Lᵢ), aus denen sich der gesamte oder ein Teil des mindestens einen Zeilenrücksprungs des Auges der Person zusammensetzt;
Berechnen mindestens eines Rückwärtssakkadenparameters (18, N_{moy}, G_{moy}, Δ) anhand der Aufzeichnungen, die den bestimmten Rückwärtssakkaden entsprechen,
wobei bei dem Verfahren der mindestens eine Rückwärtssakkadenparameter eine mittlere Anzahl von Rückwärtssakkaden (N_{moy}) über eine Vielzahl von Zeilenrücksprüngen und einen Mittelwert (G_{moy}) von Verhältnissen über die Vielzahl von Zeilenrücksprüngen kombiniert, wobei jedes Verhältnis für einen Zeilenrücksprung dem Verhältnis zwischen einer horizontalen Amplitude der zeitlich ersten Rückwärtssakkade in dem Zeilenrücksprung und einer horizontalen Gesamtamplitude von Rückwärtssakkaden, aus denen sich ein und derselbe Zeilenrücksprung zusammensetzt, entspricht.

6. Verfahren nach Anspruch 5, wobei das Verfahren den Schritt umfasst, der darin besteht, die mittlere Anzahl von Rückwärtssakkaden mit einem ersten Schwellenwert (VS) oder den Mittelwert von Verhältnissen mit einem zweiten Schwellenwert (VS) zu vergleichen.

7. Verfahren, umfassend die folgenden Schritte:
die Person (S) einem Sehtest unterziehen, der mindestens einen Zeilenrücksprung (A, B, L) mindestens eines Auges der Person beinhaltet;
während der Aufgabe Aufzeichnen der Bewegungen des Auges der Person mithilfe einer Vorrichtung zur Verfolgung von Augenbewegungen (15);
Bestimmen, mithilfe einer digitalen Verarbeitungsvorrichtung (17) und in den erhaltenen Aufzeichnungen (16), der Rückwärtssakkaden (A₁, A₂, B₁-B₄, Lᵢ), aus denen sich der gesamte oder ein Teil des mindestens einen Zeilenrücksprungs des Auges der Person zusammensetzt;
Berechnen mindestens eines Rückwärtssakkadenparameters (18, N_{moy}, G_{moy}, Δ) anhand der Aufzeichnungen, die den bestimmten Rückwärtssakkaden entsprechen,
wobei bei dem Verfahren die Bewegungen von zwei Augen der Person aufgezeichnet werden, bei denen Rückwärtssakkaden bestimmt werden, und der Rückwärtssakkadenparameter mindestens einen Parameter für die amplitudenbezogene binokulare Disjunktion (δ_{A2}, δ_{L}, Δ) umfasst, der für eine horizontale Bewegungsdifferenz zwischen den beiden Augen der Person bei mindestens einer bestimmten Rückwärtssakkade repräsentativ ist.

8. Verfahren nach Anspruch 1, bei dem der Sehtest eine Aufgabe des Lesens einer über mehrere horizontale Zeilen verteilten Symbolfolge oder eine Aufgabe des visuellen Suchens des Vorkommens eines Symbols innerhalb der Symbolfolge umfasst.

9. Verfahren nach Anspruch 1, umfassend das Vergleichen des berechneten Parameters mit mindestens einem Referenzschwellenwert.

10. Verfahren, umfassend die folgenden Schritte:
die Person (S) einem Sehtest unterziehen, der mindestens einen Zeilenrücksprung (A, B, L) mindestens eines Auges der Person beinhaltet;
während der Aufgabe Aufzeichnen der Bewegungen des Auges der Person mithilfe einer Vorrichtung zur Verfolgung von Augenbewegungen (15);
Bestimmen, mithilfe einer digitalen Verarbeitungsvorrichtung (17) und in den erhaltenen Aufzeichnungen (16), der Rückwärtssakkaden (A₁, A₂, B₁-B₄, Lᵢ), aus denen sich der gesamte oder ein Teil des mindestens einen Zeilenrücksprungs des Auges der Person zusammensetzt;
Berechnen mindestens eines Rückwärtssakkadenparameters (18, N_{moy}, G_{moy}, Δ) anhand der Aufzeichnungen, die den bestimmten Rückwärtssakkaden entsprechen, wobei man die Person (S) bei dem Verfahren einer Aufgabe des Lesens einer über mehrere horizontale Zeilen verteilten Symbolfolge und einer Aufgabe des visuellen Suchens des Vorkommens eines Symbols innerhalb einer über mehrere horizontale Zeilen verteilten Symbolfolge unterzieht; und
wobei das Verfahren das Vergleichen mindestens eines Parameters (18) unter
einer mittleren Anzahl von Rückwärtssakkaden (N_{moy}) über eine Vielzahl von Zeilenrücksprüngen bei der Leseaufgabe,
einem Mittelwert (G_{moy}) von Verhältnissen über die Vielzahl von Zeilenrücksprüngen bei der Leseaufgabe, wobei jedes Verhältnis für einen Zeilenrücksprung dem Verhältnis zwischen einer horizontalen Amplitude der zeitlich ersten Rückwärtssakkade in dem Zeilenrücksprung und einer horizontalen Gesamtamplitude der Rückwärtssakkaden, aus denen sich ein und derselbe Zeilenrücksprung zusammensetzt, entspricht,
einer mittleren Anzahl von Rückwärtssakkaden (N_{moy}) über eine Vielzahl von Zeilenrücksprüngen bei der visuellen Suchaufgabe,
einem Mittelwert (G_{moy}) von Verhältnissen über die Vielzahl von Zeilenrücksprüngen bei der visuellen Suchaufgabe, wobei jedes Verhältnis für einen Zeilenrücksprung dem Verhältnis zwischen einer horizontalen Amplitude der zeitlich ersten Rückwärtssakkade in dem Zeilenrücksprung und einer horizontalen Gesamtamplitude der Rückwärtssakkaden, aus denen sich ein und derselbe Zeilenrücksprung zusammensetzt, entspricht,
mit einem entsprechenden Schwellenwert (VS) umfasst.

11. System (1), umfassend:
- ein Testmodul (10), um eine Person (S) einem Sehtest zu unterziehen, der mindestens einen Zeilenrücksprung (A, B, L) mindestens eines Auges der Person beinhaltet;
- ein Modul (15) zur Verfolgung von Augenbewegungen, das dazu konfiguriert ist, während der Aufgabe die Bewegungen mindestens eines Auges der Person aufzuzeichnen;
- ein Modul (17) zur digitalen Verarbeitung, das dazu konfiguriert ist, in den erhaltenen Aufzeichnungen (16) Rückwärtssakkaden (A₁, A₂, B₁-B₄, Lᵢ) zu bestimmen, aus denen sich der gesamte oder ein Teil des mindestens einen Zeilenrücksprungs des Auges der Person zusammensetzt;
- ein Modul zum Berechnen mindestens eines Rückwärtssakkadenparameters (18, N_{moy}, G_{moy}, Δ), wobei der mindestens eine Rückwärtssakkadenparameter von einer Anzahl von Rückwärtssakkaden (N_{A}, N_{B}, N_{L}) abhängig ist, aus denen sich ein und derselbe Zeilenrücksprung (A, B, L) zusammensetzt, und eine mittlere Anzahl von Rückwärtssakkaden (N_{moy}) über eine Vielzahl von Zeilenwechseln umfasst, anhand der Aufzeichnungen, die den bestimmten Rückwärtssakkaden entsprechen.

## Claims

1. Method comprising the following steps:
subjecting the subject (S) to a visual test requiring at least one eye of the subject to make at least one return sweep (A, B, L);
during said task, recording the movements of the eye of the subject using a device (15) for tracking eye movements;
determining, using a digital processing device (17) and in the obtained recordings (16), return saccades (A₁, A₂, B₁-B₄, composing all or some of the at least one return sweep of the eye of the subject;
computing at least one return-saccade parameter (18, Nₘₑₐₙ, Gₘₑₐₙ, Δ) from the recordings corresponding to the determined return saccades,
method wherein the at least one return-saccade parameter is dependent on a number (N_{A}, N_{B}, N_{L}) of return saccades making up a given return sweep (A, B, L) and comprises a mean number (Nₘₑₐₙ) of returns saccades over a plurality of return sweeps.

2. Method comprising the following steps:
subjecting the subject (S) to a visual test requiring at least one eye of the subject to make at least one return sweep (A, B, L);
during said task, recording the movements of the eye of the subject using a device (15) for tracking eye movements;
determining, using a digital processing device (17) and in the obtained recordings (16), return saccades (A₁, A₂, B₁-B₄, Lᵢ) composing all or some of the at least one return sweep of the eye of the subject;
computing at least one return-saccade parameter (18, Nₘₑₐₙ, Gₘₑₐₙ, Δ) from the recordings corresponding to the determined return saccades,
method wherein at least one return-saccade parameter is dependent on a ratio (G_{A}, G_{B}, G_{L}) between a horizontal amplitude of a given return saccade in a return sweep and a total horizontal amplitude of return saccades making up the same return sweep.

3. Method according to Claim 1, wherein the given return saccade is the chronologically first saccade (A₁, B₁, in the return sweep.

4. Method according to Claim 1 or 3, wherein the at least one return-saccade parameter comprises a mean (Gₘₑₐₙ) of the ratios over a plurality of return sweeps, each ratio (G_{A}, G_{B}, G_{L}) for a return sweep corresponding to the ratio between a horizontal amplitude of the chronologically first return saccade in said return sweep and a total horizontal amplitude of return saccades making up the same return sweep.

5. Method comprising the following steps:
subjecting the subject (S) to a visual test requiring at least one eye of the subject to make at least one return sweep (A, B, L);
during said task, recording the movements of the eye of the subject using a device (15) for tracking eye movements;
determining, using a digital processing device (17) and in the obtained recordings (16), return saccades (A₁, A₂, B₁-B₄, Lᵢ) composing all or some of the at least one return sweep of the eye of the subject;
computing at least one return-saccade parameter (18, Nₘₑₐₙ, Gₘₑₐₙ, Δ) from the recordings corresponding to the determined return saccades,
method wherein the at least one return-saccade parameter combines a mean number (Nₘₑₐₙ) of return saccades over a plurality of return sweeps and a mean (Gₘₑₐₙ) of the ratios over the plurality of return sweeps, each ratio for one return sweep corresponding to the ratio between a horizontal amplitude of the chronologically first return saccade in said return sweep and a total horizontal amplitude of return saccades making up the same return sweep.

6. Method according to Claim 5, the method comprising the step consisting in comparing the mean number of return saccades to a first threshold value (VS) or the mean of the ratios to a second threshold value (VS).

7. Method comprising the following steps:
subjecting the subject (S) to a visual test requiring at least one eye of the subject to make at least one return sweep (A, B, L);
during said task, recording the movements of the eye of the subject using a device (15) for tracking eye movements;
determining, using a digital processing device (17) and in the obtained recordings (16), return saccades (A₁, A₂, B₁-B₄, Lᵢ) composing all or some of the at least one return sweep of the eye of the subject;
computing at least one return-saccade parameter (18, Nₘₑₐₙ, Gₘₑₐₙ, Δ) from the recordings corresponding to the determined return saccades,
method wherein the movements of both eyes of the subject are recorded, from which movements return saccades are determined, and the return-saccade parameter comprises at least one amplitudewise binocular disconjugacy parameter (δ_{A2}, δ_{L}, Δ) representative of a difference in horizontal movement between the two eyes of the subject during at least one determined return saccade.

8. Method according to Claim 1, wherein the visual test comprises a reading task in which a sequence of symbols distributed over a plurality of horizontal lines is read or a visual search task in which occurrences of a symbol within the sequence of symbols are sought.

9. Method according to Claim 1, comprising comparing the computed parameter with at least one reference threshold value.

10. Method comprising the following steps:
subjecting the subject (S) to a visual test requiring at least one eye of the subject to make at least one return sweep (A, B, L);
during said task, recording the movements of the eye of the subject using a device (15) for tracking eye movements;
determining, using a digital processing device (17) and in the obtained recordings (16), return saccades (A₁, A₂, B₁-B₄, Lᵢ) composing all or some of the at least one return sweep of the eye of the subject;
computing at least one return-saccade parameter (18, Nₘₑₐₙ, Gₘₑₐₙ, Δ) from the recordings corresponding to the determined return saccades,
method wherein the subject (S) is subjected to a reading task in which a sequence of symbols distributed over a plurality of horizontal lines is read and to a visual search task in which occurrences of a symbol within a sequence of symbols distributed over a plurality of horizontal lines are sought; and
the method comprising comparing at least one parameter (18) among
a mean number (Nₘₑₐₙ) of return saccades over a plurality of return sweeps during the reading task,
a mean (Gₘₑₐₙ) of the ratios over the plurality of return sweeps during the reading task, each ratio for one return sweep corresponding to the ratio between a horizontal amplitude of the chronologically first return saccade in said return sweep and a total horizontal amplitude of the return saccades making up the same return sweep,
a mean number (Nₘₑₐₙ) of return saccades over a plurality of return sweeps during the visual search task,
a mean (Gₘₑₐₙ) of the ratios over the plurality of return sweeps during the visual search task, each ratio for one return sweep corresponding to the ratio between a horizontal amplitude of the chronologically first return saccade in said return sweep and a total horizontal amplitude of the return saccades making up the same return sweep,
to a corresponding threshold value (VS).

11. System (1) comprising:
- a testing module (10) for subjecting the subject (S) to a visual test requiring at least one eye of the subject to make at least one return sweep (A, B, L);
- a module (15) for tracking eye movements, said module being configured to record, during said task, the movements of at least one eye of the subject;
- a digital processing module (17) configured to determine, in the obtained recordings (16), return saccades (A₁, A₂, B₁-B₄, Lᵢ) composing all or some of at least one return sweep of the eye of the subject;
- a module for computing at least one return-saccade parameter (18, Nₘₑₐₙ, Gₘₑₐₙ, Δ), where the at least one return-saccade parameter is dependent on a number of return saccades (N_{A}, N_{B}, N_{L}) making up a given return sweep (A, B, L) and comprises a mean number (Nₘₑₐₙ) of return saccades over a plurality of return sweeps, from the recordings corresponding to the determined return saccades.
